# EUROPEAN PATENT APPLICATION

(11) **EP 0 633 279 A1**
(43) Date of publication of application: **11.01.1995**
(21) Application number: 94110629.6
(22) Date of filing: 08.07.1994
(51) Int. Cl.: C08G 65/32, C11D 1/12, C11D 1/08, C08F 283/06

(54) **Preparation of ether carboxylates**

(30) Priority: 10.07.1993 GB 9314277
(71) Applicant: ALBRIGHT & WILSON UK LIMITED, Oldbury, Warley, West Midlands B68 0NN (GB)
(72) Inventor: Chadwick, Ruth Emma, Warley, West Midlands B68 1UL (GB); Phillips, Bryn Morris, Kidderminster, Worcestershire DY10 4AX (GB)
(74) Representative: Savidge, Roger Gordon Madgwick

(57) **Abstract**

Ether carboxylates are prepared by reacting a compound having a polyoxyalkylene chain with a water soluble salt of an unsaturated carboxylic acid in aqueous solution in the presence of a free radical initiator.

## Description

The present invention relates to a novel method for the preparation of polycarboxylates, more specifically to a novel method for the preparation of adducts of alkylene oxide polymers with unsaturated carboxylic acids. The invention is of particular value for the manufacture of alkyl or alkenyl ether polycarboxylic acid salts, hereinafter collectively referred to as "AEPs". The invention also provides novel AEPs which can be prepared by the novel method.

### Technical Background

AEPs have been reported as mild anionic surfactants which are capable of sequestering calcium ions, thus being able to act as 'self-building' surfactants.

However, hitherto, no totally satisfactory method for the preparation of AEPs in a commercially acceptable yield, free from by-products, has been disclosed. The only known preparative methods typically produce AEPs of variable character, which require neutralisation prior to use and which may be hydrolytically unstable. In particular, on neutralisation to alkaline pH, e.g. 7 or above, they typically form viscous mesophases which often undergo phase separation on dilution or which do not produce clear solutions. We have discovered that the products hitherto referred to in the literature as 'AEPs' are in fact complex mixtures containing from 25 to 50% by weight of esters, and numerous undesirable by-products formed by side reactions of the ester. Low yields of the unesterified AEP thus result, often coupled with unreproducible results due to the complex side reactions possible. It has not proved possible to purify or to characterise the product by conventional methods.

To date the only commercially acceptable surfactants that have been made by the known method are based on feedstocks having relatively short alkyl chains (e.g. 8 to 12C and having an average less than 11) and high propylene oxide (.e.g. more than 10% molar of the total alkylene oxide). Such products are adapted to a limited range of industrial uses. One such use is the levelling of dyes in textile treatment, where the use of AEPs is known (W0 9100318). It has not proved possible to make acceptable products from feedstocks which are more adapted to detergent or toiletry applications, such as C₁₂₋₁₄ alcohols. Moreover propylene oxide based polyalkoxylates are considered undesirable on environmental grounds because they are less biodegradable than ethylene oxide based products. The known technology, however, has not been found capable in practice of providing commercially viable products based on ethylene oxide. The known method has only been applied to alkoxylated alcohols and is generally not applicable to other alkoxylates, such as alkyl polyalkyleneoxy carboxylic acids. The only derivatives of AEPs have been compounds such as the sulphate prepared by sulphating the complex mixture of AEP and esters. This produces a similarly complex mixture with relatively little of the desired carboxylated ether sulphate.

### Prior Art

The production of AEPs by the direct free radical addition reaction of an olefinically unsaturated carboxylic acid, e.g. maleic, fumaric or itaconic acid, to the backbone of a C₆-C₁₈ polyoxyalkylated (propoxylated and/or ethoxylated) alcohol using a peroxy type free radical initiator is described for example in EP O 129 328 and W0 9000538. The use of solvents as the reaction medium is not required (other than optionally to reduce the viscosity in the case of an exceptionally viscous ethoxylated alcohol). The AEP is produced in the acid form, the salts being obtained by subsequent neutralisation of the acid moiety with neutralising agents such as alkali metal hydroxides. Only propylene oxide based products are exemplified.

This method however, gives a product which tends to hydrolyse on neutralisation giving rise to cloudiness and poor physical properties, which we believe, are due to the formation of a high level of undesirable esterified product, and other by-products.

US 4,533,486 describes sulphated AEPs produced by sulphation of products of the type obtained via EP 0 129 328. However, the levels of esters and other by-products are again unacceptably high, with the products often proving unreproducible. US 4,827,028 describes the preparation of epoxycapped AEPs following the method disclosed in US 4,533,486. As in the previous preparations, unacceptable levels of ester formation and other by-products result.

The production of monoether and polyether polyol polycarboxylates, and their polyurethane prepolymer derivatives is described in EP 0 119 349. Again unacceptable levels of by-products are produced, which cannot readily be separated form the carboxylated polyether.

### The Problem

It is an object of this invention to provide polycarboxylated polyalkoxylates, including AEPs, containing reduced levels of by-products, especially esterified by-products, compared with the products prepared according to the prior art.

It is a further object of this invention to provide AEP's which upon neutralisation or dilution do not produce hazy or phase separated solutions as do many AEPs produced according to the prior art, but which are clear homogenous single phase solutions upon dilution. One particular object for a preferred embodiment is to provide AEPs which give clear solutions at high concentrations in water, at alkaline pH.

It is a further object of the invention to provide novel polycarboxylated polyalkoxylates not hitherto obtainable, or at least not previously obtained, using the conventional technology.

It is a further object of this invention to provide a one step method for the preparation of AEPs and other ether polycarboxylate salts which preferably provides a product having lower levels of ester and other by-products, and/or with improved reproducibility and/or in an improved yield, compared with the prior art.

It is a further object of this invention to provide an AEP product suitable for use in toiletries, and detergents including laundry detergents, cleaning agents for use in oilfield and drilling operations, hard surface cleaners, dishwashing and/or car washing products, and also for use in a range of solid suspending systems such as drilling muds and other functional fluids, pesticide formulations and for suspending dyes or pigments.

It is also an object of this invention to provide biogradable polycarboxylated polyalkoxylates based on ethylene oxide and substantially free from oxypropylene residues.

Another object is to provide AEPs wherein the alkyl or alkenyl group or groups have more than 10, e.g. 10 to 20 especially 12 to 14 carbon atoms and preferably an average greater than 11.

### The Invention

We have now discovered that polycarboxylated polyalkoxylates and their sulphate derivatives may be prepared by the reaction of an ethoxylated and/or propoxylated alcohol with a water soluble, e.g. alkali or alkaline earth metal salt of an unsaturated carboxylic acid, in aqueous solution in the presence of a free radical initiator, giving products of improved yield and reduced impurity levels compared with the essentially anhydrous reactions with free carboxylic acids which have been used hitherto. We have discovered that the method provides products which give clear solutions on neutralisation which remain clear and homogenous on dilution and which are well adapted to detergent and toiletry use.

We have further discovered that the above reaction conditions are applicable to the preparation of a range of novel adducts of unsaturated carboxylic acids with compounds containing polyalkylene oxide chains.

### Statement of Invention

Our invention provides a method for the manufacture of polycarboxylates which comprises reacting (i) a compound having at least one polyalkyleneoxy chain and (ii) a water soluble salt of an unsaturated carboxylic acid, in aqueous solution in the presence of a free radical initiator.

According to a second embodiment of the present invention a method for the preparation of AEPs is provided in which said compound (i) is an alkyl polyalkoxylate, preferably a polyethoxylate.

According to a third embodiment the present invention provides a method for the manufacture of AEPs which method comprises reacting: (i) an alkoxylated aliphatic alcohol, wherein said alcohol has from 6 to 25 carbon atoms and is alkoxylated with from 1 to 50 ethyleneoxy and/or propyleneoxy groups; with (ii) a water soluble salt of an unsaturated carboxylic acid having from 3 to 6 carbon atoms; in an aqueous reaction medium, in the presence of a free radical initiator.

Preferably the invention comprises preparing polycarboxylated polyalkoxylates of general formula (I): 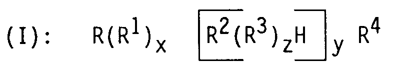
in which R is a straight or branched chain alkyl, alkaryl or alkenyl group or straight or branched chain alkyl or alkenyl carboxy group, having, in each case, up to 27, typically from 6 to 25 carbon atoms, or hydrogen, each R¹ is an OCH₂CH₂ or an OCH(CH₃)CH₂ group, each R² is an OC₂H₃ or OC₃H₅ group, each R³ is a C(R⁵)₂C(R⁵)₂ group, wherein from 1 to 4, preferably 2, R⁵ groups per R³ group are C0₂A groups, each other R⁵ group being a C₁₋C₂ alkyl, hydroxy alkyl or carboxyalkyl group or, preferably H, R⁴ is OH, SO₄A, SO₃A, OR, sulphosuccinyl, OCH₂CO₂A, or R⁶₂NR⁷, R⁶ is a C₁-C₄ alkyl or hydroxyalkyl group, R⁷ is a C₁-C₂₀ alkyl group, a benzyl group, a CH₂CO₂A, or -> 0 group or PO₄A₂, A is a cation capable of forming water soluble salts of said carboxylic acid such as an alkali metal or alkaline earth metal, each z is from 1 to 5 preferably 1, y is at least 1 and (x+y) has an average value of from 1 to 50, wherein the R¹ and R² groups may be arranged randomly or in any order along the polyalkoxylate chain; which method comprises, reacting (i) a compound of the formula R(R¹)(x+y)R⁴ wherein R, R¹ and R⁴ are as hereinbefore defined with (ii) a water soluble salt of an unsaturated carboxylic or polycarboxylic acid C(R⁵)₂ = C(R⁵)₂ where each R₅ is as defined above, in aqueous solution in the presence of a free radical initiator, at a temperature sufficient to produce said polycarboxylate.

According to a further embodiment, the invention provides the use of the polycarboxylates according to the invention, e.g. AEPs and their derivatives, in toiletries, hard surface cleaners and other detergent compositions.

According to a further embodiment our invention provides an improved AEP which is substantially free from esters and which is soluble to give clear stable homogenous solutions at all concentrations up to 50% at a pH between 7 and 8. In particular the invention provides such AEPs having an average alkyl chain length of from 10 to 18 carbon atoms, and a polyether chain consisting of from 2 to 10 ethyleneoxy groups.

According to a further embodiment the invention provides a novel surfactant having the formula (I) above wherein R is a C₆ to ₂₀ alkyl or alkenyl group or C₁₀ to ₂₀ alkyl or alkenyl carboxy group and R⁴ is a CH₂CO₂A or R⁶₂ NR⁷ group where R⁶, R⁷ and A have the same significance as before.

According to a further embodiment the invention provides an N-C₆₋₂₀ alkyl or alkenyl 2 to 20 mole polyoxyalkylene-N,N-dialkyl amine N-oxide poly carboxylate.

According to a further embodiment the invention provides an N-C₆₋₂₀ alkyl or alkenyl 2 to 20 mole polyoxyalkylene-N-carboxymethyl polycarboxylate.

According to a further embodiment our invention provides a method for the production of polycarboxylated ether sulphates which comprises reacting a water soluble alkyl or alkenyl polyoxyalkylene sulphate with a water soluble salt of an unsaturated carboxylic acid in aqueous solution in the presence of a free radical initiator.

AEP products provided according to the present invention contain a lower level of esters and other by-products, and are obtained in higher yields and with a greater purity and improved reproducibility than corresponding products previously obtained by known methods. The AEP products produced according to the method of the present invention also typically exhibit good solubility, foaming and hydrolytic stability characteristics. In particular, they do not exhibit , or are substantially less prone to, the phase instability problems upon dilution in alkaline media associated with many of the AEP's prepared according to the prior art.

### The Polyalkoxylate Reagent (i)

Polyalkoxylates (i) suitable for use according to the present invention are those having an alkoxylated chain with an average value of between 1 and 50 alkoxy units, preferably between 1 and 50 ethoxy and/or propoxy units. Preferred are polyethoxylates such as polyethylene glycol or less preferably polyethylene/polypropylene glycol copolymers containing up to 40% molar of propylene oxy residues, e.g. 0 to 20% molar preferably less than 10% molar. The polyalkyleneoxy chain may be terminated at one or both ends by organic or inorganic groups, including C₁ to ₂₂, preferably C₆ to ₂₀, especially C₁₀ ₂₀ alkyl or alkenyl groups or alkyl or alkenyl carboxy groups (R- or RCO-) or alkylaryl groups including alkylphenyl groups, alkylene carboxylate [(CH₂)ₙ CO₂A] groups, a sulphosuccinyl group, glycoside, polyglycoside, alkyl glycoside or alkyl polyglycoside residues including sorbitan, sorbitan ester, fructoside or glucoside residues, or alkyl glucoside residues, glyceride residues including mono or di fatty acid glyceride residues (e.g. ROCH₂CHOHCH₂- or ROCH₂ROCH CH₂), sulphate groups, phosphate or phosphate ester groups, amino groups including trialkyl ammonium (e.g. di C₁ to ₄ alkyl such as dimethyl mono C₁₆ to ₂₀ alkyl ammonium), dialkyl benzylammonium (e.g. di C₁ to ₄ alkyl such as dimethyl benzyl), dialkyl amine oxide, (eg. di C₁ to ₄ alkyl such as dimethylamine oxide) dialkylglycine (e.g. di C₁-₄ alkyl such as dimethyl glycine) and alkyl aminomethylene groups, as in ethoxylated ethanolamides e.g. ethoxylated coconut mono-or di-ethanolamide.

Suitable polyalkoxylates, for use as (i) according to the present invention therefore include those of general formula (II):-

(II) R(R¹)_{(x+y)}R⁴

Wherein R,R¹ and R⁴ are as hereinabove defined in relation to formula (I), and (x+y) has an average value of from 1-50. Suitable polyalkoxylated compounds for use according to the present invention include fatty alcohol alkoxylates, fatty acid alkoxylates, alkylphenyl alkoxylates, fatty acid alkylolamide ethoxylates, including ethoxylated mono and dialkylolamides, alkoxylated quaternary ammonium salts for example ethoxylated amine oxides and ethoxylated betaines and alkyl polyethoxy sulphates and sulphonates.

Particularly preferred are C₁₀ to ₂₀ alkyl or alkenyl 2 to 20 mole ethoxylates, N-C₁₀ to ₂₀ alkyl or alkenyl 2 to 20 mole polyoxyethyene-N,N-di C₁ to ₄ alkylamine-N-oxides and N-C₁₀ to ₂₀ alkyl or alkenyl 2 to 20 mole polyoxyethylene-N carboxymethylamines optionally with one or two additional N-alkyl substituents.

In the case of an alkoxylated alcohol feedstock, the use of an alkoxylate (i) containing a low level of free alcohol, e.g. less than 5%, preferably less than 2%, more preferably less than 1%, most preferably less than 0.5% by weight, based on the total weight of (i), increases the water solubility of the alkoxylate compared to samples containing higher levels of free alcohol, consequently reducing the tendency of the AEP to become cloudy upon dilution, an important property in detergent formulations. Low levels of free alcohol may be achieved by using more highly alkoxylated starting materials or by the use of selective alkoxylation catalysts and/or by stripping.

The alkoxylate (i) may for example be stripped using a wiped film evaporator, prior to the addition reaction, to minimise the level of free alcohol present.

### The Carboxylate Reagent (ii)

The cation A is usually sodium, but may also be potassium or a sodium/potassium mixture. Other alkali metals such as lithium, rubidium or caesium are technically operable but unlikely to be selected on economic grounds. A may alternatively or additionally comprise ammonium or an amine such as isopropylamine or mono, di or triethanolamine or methyl pyridinium or quinolinium. Alkaline earth metals such as zinc, calcium or magnesium are less preferred. Where reagents (i) and (ii) both comprise acidic groups, the counter-ions A may be the same or different.

The carboxylate (ii) may be added preferably as the sodium or potassium salt or alternatively the salt may be generated in situ by the neutralisation of the appropriate acid with a suitable amount of an acid-neutralising agent, e.g. an alkali metal or alkaline earth metal hydroxide.

The unsaturated carboxylate is preferably a salt of an unsaturated di- or polycarboxylic acid. Preferably the double bond is adjacent to at least one carboxyl group. We prefer that the carboxylate has less than six e.g 3 to 5 carbon atoms. The preferred carboxylate is maleate. Other suitable carboxylates include fumarate, acrylate, itaconate, methacrylate, crotonate, isocrotonate, angelate, tiglate, and mesaconate.

The proportion of carboxylate (ii) may conveniently be in the range of from 0.2:1 to 5:1 molar, based on the number of moles of alkoxylate monomer units in the polyoxyalkylene chains, or 0.02:1 to 20:1 molar based on the moles of the polyalkoxylate (i) e.g. 0.1:1 to 5:1.

### The Initiator

The initiator may comprise a peroxy compound. Where the reagents (i) and (ii) are fully soluble we prefer to use an inorganic initiator especially hydrogen peroxide, or, less preferably a persulphate or percarbonate. Where the reagent (i) is sparingly soluble we prefer to use an organic initiator e.g one which is soluble in reagent (i) such as an organic hydroperoxide or peroxide.

The organic hydroperoxide or peroxide based initiator is preferably tertiary butyl perbenzoate. It may alternatively be, for example, tertiary butyl monopermaleate, di(tertiary butyl) peroxide, dilauroyl peroxide, tertiary butyl hydroperoxide, tertiary butyl peroxybenzoate, or tertiary butyl peroxy-2-ethylhexanoate.

The initiator may be added as a single addition to the reaction medium, or in aliquots, or continuously over the reaction period. Typically, the quantity of initiator used is 0.01-15% w/w, preferably 1-10 % w/w based on the total weight of the reactants, although the amount required is also governed by the reaction conditions.

It may also be possible in some cases to initiate the reaction photochemically e.g. using ultra violet or other energetic radiation or to use irradiation in conjunction with a chemical source of free radicals.

### Reaction Medium

Water typically constitutes 20-80% preferably 30-70% by weight of the total weight of the reaction medium. The presence of water miscible solvents in the reaction medium is not normally necessary or desirable but may be advantageous where the solubility of the reagent (i) is low or the medium is unduly viscous. Water miscible mono or polyhdyric alcohols such as methanol, ethanol, isopropanol, ethylene glycol, glycerol, or propylene glycol, may for example be used in small amounts e.g. up to 10% by weight of the reaction mixture. In general glycol ether solvents will constitute part of the reagent (i) and should not be added unless so required.

### Reaction Conditions

The reaction medium is preferably deoxygenated, for example by bubbling nitrogen through the reaction medium. Upon completion of the deoxygenation, the reaction vessel may be heated to a sufficient temperature to activate the free radical initiator.

The pH of the reaction medium is typically in the range pH 5-10, preferably above 5.5, e.g. pH 6-9.5 especially 6.5 to 9.

Reaction times may typically be from 0.1 to 48 hours, preferably 2 to 26 hours, such as 7 to 31 hours, e.g. 10 to 20 hours, dependent upon temperature and reagents employed.

The reaction temperature may, if the initiator is sufficiently active, be ambient but is preferably elevated, e.g. 60 to 280°C. The reaction may be carried out under reflux or under pressure in an autoclave, and the temperature maintained until the reaction is substantially complete.

The reaction may carried out batch wise, continuously or semi continuously.

The AEP may be purified by removing any lower boiling fractions such as may have been formed from decomposition of the initiator, by any conventional purification technique, e.g. by distillation. Excess carboxylic acid may be removed by crystallisation, e.g. evaporating the reaction product until turbidity is observed, cooling and filtering off crystals of acid.

The product may be treated with a suitable bleaching agent, preferably hydrogen peroxide, if required to provide a lighter colour. Other suitable bleaching agents include peroxides, percarbonates and perborates, chlorine dioxide and sodium borohydride.

### Structured Surfactants

The polycarboxylates according to our invention are especially useful in structured surfactant systems. They permit stable structured surfactants to be prepared at higher concentrations than have hitherto been conventional.

Structured surfactants are liquid systems in which surfactant mesophases such as the lamellar G-phase, or interspersions of mesophase and aqueous solutions, usually containing dissolved electrolyte, give shear dependant viscosity and, typically, a yield point. This enables the systems to be readily pourable and yet to maintain even coarse particulate solids in suspension indefinitely. These systems find applications in a wide variety of fields where a particulate solid is required to be suspended in a pourable liquid medium including built liquid detergents, scouring preparations, oilwell drilling muds, toiletries and pesticide formulations.

The most commonly used systems comprise spherulites, usually about 0.1 to 20 microns in diameter, and formed from concentric shells each comprising a bilayer of surfactant molecules alternating with the aqueous medium. The spherulites can pack together within the continuous aqueous phase to form a structure which has sufficient rigidity to prevent the movement of particles suspended therein but is weak enough to break down under the shear stresses applied when the composition is poured, resulting in a mobile composition.

Structured surfactants may be formed spontaneously by some surfactants at certain concentrations, but normally result from the interaction of surfactants with dissolved, surfactant-desolubilising electrolytes.

Some methods of obtaining structured surfactants are described, for example, in US 4515704, US 4618446 and EP O530708.

### Detergent Compositions

The surfactants of our invention are suitable for use as detergents, functioning as both surfactants and builders. Aqueous solutions containing concentrations up to 60% by weight, or in some cases more, of the surfactants may for example be used.

The invention further provides formulated detergents containing the surfactants.

The detergent compositions of our invention may be solid powders or bars, clear liquids or structured liquids which may contain suspended solid.

The compositions of the present invention preferably contain at least 5% by weight of surfactant based on the total weight of the composition, more preferably at least 10%, most preferably at least 20%, especially more than 25%, e.g. more than 30%. It is unlikely in practice that the surfactant concentration will exceed 80% based on the weight of the composition.

The detergent compositions of the invention may comprise surfactants in addition to AEPs including anionic, cationic, non-ionic, amphoteric or zwitterionic species or mixtures thereof.

The anionic surfactant may comprise alkyl ether sulphate which is preferably the product obtained by ethoxylating a natural fatty or synthetic C₁₀₋₂₀ (e.g. a C₁₂₋₁₄) alcohol with from 1 to 20, preferably 2 to 10 e.g. 3 to 4 ethyleneoxy groups, optionally stripping any unreacted alcohol, reacting the ethoxylated product with a sulphating agent and neutralising the resulting alkyl ether sulphuric acid with a base. The term also includes alkyl glyceryl sulphates, and random or block copolymerised alkyl ethoxy/propoxy sulphates.

C₆₋₂₀ alkyl benzene sulphonates can be used as components of our surfactant mixture. The surfactant may also comprise, for example, C₁₀₋₂₀ eg. C₁₂₋₁₈ alkyl sulphate.

The surfactant may preferably comprise a C₈₋₂₀ e.g. C₁₀₋₁₈ aliphatic soap. The soap may be saturated or unsaturated, straight or branched chain.

Preferred examples includes dodecanoates, myristates, stearates, oleates, linoleates, linolenates and palmitates and coconut and tallow fatty acids and their water soluble salts. Where foam control is a significant factor we particularly prefer to include soaps eg, ethanolamine soaps and especially triethanolamine soaps, which have been found to give particularly good cold storage and laundering properties.

According to a further embodiment, the soap and/or carboxylic acid is preferably present in a total weight proportion, based on the total weight of surfactant, of at least 20% more preferably 20 to 75% most preferably 25 to 50%, e.g. 29 to 40%.

The detergent compositions of the invention may include other anionic surfactants, such as olefin sulphonates, paraffin sulphonates, taurides, isethionates, ether sulphonates, aliphatic ester sulphonates eg, acyl glyceryl sulphonates, alkyl ether carboxylates, sulphosuccinates or sulphosuccinamates. Preferably the other anionic surfactants are present in total proportion of less than 45% by weight, based on the total weight of surfactants, more preferably less than 40% most preferably less than 30% e.g. less than 20%.

The cation of the anionic surfactant is typically sodium but may alternatively be potassium, lithium, calcium, magnesium, ammonium, or alkyl ammonium having up to 6 aliphatic carbon atoms including isopropylamine, monethanolammonium, diethanolammonium, and triethanolammonium. Ammonium and ethanolammonium salts are generally more soluble than the sodium salts.

The detergent compositions of the invention preferably contain one or preferably more, non-ionic surfactants. These preferably comprise alkoxylated, e.g. exthoxylated, C₈₋₂₀ preferably C₁₂₋₁₈ alcohols, alkoxylated with 2 to 20 especially 2.5 to 15 alkyleneoxy groups.

The alcohol may be fatty alcohol or synthetic e.g. branched chain alcohol.

Preferably the non-ionic component has an HLB of from 6 to 16.5, especially from 7 to 16 e.g. From 8 to 15.5. We particularly prefer mixtures of two or more non-ionic surfactants having a weighted mean HLB in accordance with the above values.

Other alkoxylated non-ionic surfactants which may be present include C₆₋₁₆ alkylphenol alkoxylates, alkoxylated fatty acids, alkoxylated amines, alkoxylated alkanolamides and alkoxylated alkyl sorbitan and/or glyceryl esters. The alkylene oxide in the non-ionic alkoxylates is, in each case, preferably ethylene oxide, but may alternatively be propylene oxide or a mixture of ethylene oxide and propylene oxide.

Other non-ionic surfactants which may be present include amine oxides, fatty alkanolamides such as coconut monoethanolamide, and coconut diethanolamide, alkylpolyglycosides and alkylaminoethyl fructosides and glucosides.

The proportion by weight of non-ionic surfactant is preferably at least 2% and usually less than 40% more preferably less than 30% eg, 3 to 25% especially 5 to 20% based on total weight of surfactant.

The detergent formulations of the invention may optionally comprise minor amounts of amphoteric and or cationic surfactants, for example betaines, imidazolines, amidoamines, quaternary ammonium surfactants and especially cationic fabric conditioners having two long chain alkyl groups, such as tallow groups.

Detergent formulations according to our invention contain builders in addition to the polycarboxylate of the invention. "Builder" is used herein to mean a compound which assists the washing action of a surfactant by ameliorating the effects of dissolved calcium and/or magnesium.

Generally builders also help maintain the alkalinity of wash liquor. Typically builders include sequestrants and complexants such as sodium tripolyphosphate, potassium pyrophosphate, trisodium phosphate, sodium ethylenediamine tetracetate, sodium citrate or sodium nitrilotriacetate, ion exchangers such as zeolites and precipitants such as sodium or potassium carbonate and such other alkalis as sodium silicate. The polycarboxylate of the invention also contributes to the total builder. The preferred builders are sodium tripolyphosphate and zeolite . The builder may typically be present in concentrations up to 50% by weight of the composition e.g. 15 to 30%.

Where appropriate, detergent compositions of our invention may optionally contain small amounts of hydrotropes such as sodium xylene sulphonate, sodium toluene sulphonate or sodium cumene sulphonate, e.g in concentrations up to 5% by weight based on the total weight of the composition, preferably not more than 2%, e.g. 0.1 to 1%. Hydrotropes tend to break surfactant structure and cause instability of structured surfactants. They also add to the cost of the formulation, without improving its performance and it is therefore preferred to use the minimum amounts consistent with effectiveness. Hydrotropes are primarily useful for increasing the water solubility of less soluble components and for lowering the viscosity of liquid formulations, especially if the detergent contains surfactants in structured form. Hydrotropes are not normally required in formulations of the present invention.

If required, liquid detergent compositions of the invention may contain solvents. However, like hydrotropes, solvents tend to break surfactant structure, and they add to the cost of the formulation. They are moreover undesirable on environmental grounds. We therefore prefer that the compositions contain less than 6%, more preferably less than 5% most preferably less than 3%, especially less than 2%, more especially less than 1%, e.g. less than 0.5% by weight of solvents such as water miscible alcohols or glycols, based on the total weight of the composition.

The composition may contain stain removers. These may include phosphonates, enzymes and/or bleaches. Phosphonates include acetodiphosphonates, amino tris (methylene phosphonates), ethylenediamine tetrakis (methylene phosphonates), diethylenetriamine pentakis (methylene phosphonates) and higher members of the series. Enzymes include proteases, lipases, amylases, cellulases and/or decarboxylases. Bleaches include oxidising bleaches (where chemically compatible with the formulation) such as perborates, peroxides, percarbonates and hypochlorates, and photoactivated bleaches.

The detergent compositions of our invention preferably also comprise conventional detergent additives such as antiredeposition agents (typically sodium carboxymethyl cellulose), optical brighteners, sequestrants, antifoams, such as silicone antifoams, enzyme stabilisers such as borax, polyvinyl alcohol or polyvinyl pyrrolidone, preservatives, dyes, pigments, perfumes, fabric conditioners, e.g. cationic fabric softeners such as ditallowyl dimethyl ammonium salts, or clays such as bentonite, opacifiers and/or bleach activators such as tetra acetylethylene diamine.

Solid detergent compositions of our invention usually comprise inert fillers such as sodium sulphate to promote the formation of a free flowing powder. The powder is usually prepared by spray drying a slurry of the various components.

Generally all conventional detergent additives which are compatible with the detergent composition may be included.

### Toiletries

The polycarboxylated polyalkoxylates according to our invention may be used in toiletries, including shampoos, liquid or solid hand washing formulations, creams, lotions, balms, ointments, antiseptics, dentifrices and styptics. They may be used in structured systems to suspend exfoliants including talc, clays, polymer beads, sawdust, silica, seeds, ground nutshells and dicalcium phosphate, pearlisers such as mica, glycerol mono-or di-stearate or ethylene glycol mono-or di-stearate, natural oils, such as coconut, evening primrose, groundnut, meadow foam, apricot kernel, avocado, peach kernel or jojoba oils, synthetic oils such as silicone oils, vitamins, anti-dandruff agents such as zinc omadine, and selenium disulphide, proteins, emollients such as lanoline or isopropylmyristate, waxes such as carnauba wax and sunscreens such as titanium dioxide and zinc oxide.

### Other Structured Systems

The polycarboxylated polyalkoxylates e.g. AEPs according to our invention may also be used in surfactant systems to suspend abrasives such as talc, silica, calcite or coarse zeolite to give hard surface cleaners. They may be used to suspend pesticides, to provide water-dispersible, pourable compositions containing water insoluble pesticides, without the hazards of toxic dust or environmentally harmful solvents. They can suspend dyes and pigments to provide dyebath concentrates or printing inks, and they are useful in pharmaceuticals, biocides, or as drilling muds, for drilling oil wells and boreholes. Such muds usually contain suspended shale and/or weighting agents such as sodium chloride, galena, calcite, magnetite, barite or haematite. This surfactant may also be used in cutting fluids and lubricants.

### Other Surfactant Applications

The surfactant products of our invention may be used for a variety of other applications. For example they are useful as emulsifiers, wetting agents, foam controlling agents and dispersants. Subject to toxicity constraints which may apply to certain of the products, they may be used in the food industry, in latex emulsions, paints, disinfectants, bleaches, adhesives, carpet shampoos, resin emulsions, emulsion polymerisation, the production of foamed polymers, such as polyurethanes, as lubricants in plastics extrusion and rubber moulding, in photographic emulsions, as wetting agents in the fractionation of fats and fatty acids, as dispersants in textile print pastes, and collectors in ore floatation and in a wide range of industrial process applications.

### Water Treatment

Products according to our invention are especially useful in water treatment. In addition to the surfactant uses noted above the products are of value in preventing or inhibiting corrosion and scale formation in aqueous systems such as cooling water used in heat exchangers and cooling towers, water in central heating systems, water injected into oil wells to recover reserves or clean boreholes and in desalination.

Threshold concentrations of 0.1 to 100 ppm of the products of our invention have in some cases been found sufficient to effect substantial improvements in corrosion and/or scale formation. The non-surfactant products of our invention, e.g. those lacking a C₆ to ₂₅ alkyl group such as polycarboxylated polyethylene glycol, are particularly preferred in such applications.

The products can be formulated with other water treatment agents including other corrosion and/or scaling inhibitors such as phosphono carboxylic acids and/or polymaleic acid, biocides, dispersents, deflocculants, synergists and antifoams.

The invention will be further illustrated by way of the following examples:

### Example 1

### The Manufacture of C₁₂-C₁₄ Alkyl Ether Polycarboxylates

To di-sodium maleate monohydrate (106.8g, 0.6 mol) dissolved in water (260g), ethoxylated C₁₂₋₁₄ alcohol (3 E0) (111.2g, 0.3 mol) was added, and N₂ passed through the resulting mixture for 1 hour at 40^{o}C.

The deoxygenated mixture was heated to reflux and the initiator, tertiary butyl perbenzoate (57.3g, 0.3 mol) was added slowly in a total of 4 aliquots over 12 hours (3 aliquots in the 1st 4 hours, the remaining aliquot after a further 8 hours).

The mixture was refluxed for a further 4 hours, after which time the lower boiling fraction (2-methyl-2-propanol) from decomposition of the initiators, was distilled off.

The unbleached product was an orange liquid produced in yield - 77%.

Half of the remaining mixture (166g) was treated with 30 vol hydrogen peroxide to produce a light yellow clear mobile liquid.

The product was identified by NMR/LCMS.

### Product Analysis

| | |
|---|---|
| Maleate/dicarboxylate content - (Ion Chromatography) | 17.9% before. |
| | 3.0% after. |
| Ethoxylate content = (High Pressure Liquid Chromatography) | 20.0% before. |
| | 4.75% after. |

The product was compared with that obtained by reacting the same ethoxylate with maleic acid under anhydrous conditions as taught in the prior art. The comparative product was a viscous brown oil which was substantially insoluble in water. On neutralising with sodium hydroxide a viscous brown pasty composition was obtained at 50% concentration. On dilution to 20% the composition went opaque to form a light brown, mud coloured liquid which slowly separated into two layers on standing. In contrast the product of Example 1 was a clear yellow mobile liquid at 50% which diluted to a clear, very pale yellow liquid at 20%.

### Example 2

### The Manufacture of C₁₂-C₁₄ (Alkyl Ether Polycarboxylates)

Ethoxylated C₁₂₋₁₄ alcohol (3EO) (111.1g, 0.3mol) and distilled water (80g) were added to a suitable reaction vessel and dexoygenated by passing N₂ through the mixture for 1 hour at 50°C. The deoxygenated mixture was heated to reflux temperature.

To the refluxing mixture over a period of 5 hours an aqueous solution of maleic acid (104.5g, 0.9mol) and sodium hydroxide (72.2g, 0.3mol) was added in 3 aliquots. Three of a total of five aliquots of an aqueous solution of tertiary butyl perbenzoate (41.7g, 0.2mol) were added simultaneously with the acid/hydroxide aliquots. The remaining 2 aliquots of initiator were added over a further 10 hours. After a total refluxation time of 23 hours, a further addition of tertiary butyl perbenzoate was made (25g, 0.13mol) and the reaction allowed to continue until the total reaction time had reached 31 hours.

The unbleached product (620g), was an orange liquid of general formula (I), obtained in high yield (92%).

Treatment with 30 vol hydrogen peroxide, produced an AEP product of pale yellow colour. The lower boiling fraction (2-methyl-2-propanol) from decomposition of the initiator was removed by distillation.

The product was identified by NMR/LCMS.

### Product Analysis

| | |
|---|---|
| Maleate content (Ion Chromatography) | < 4.5% |
| Ethoxylate content (High Pressure Liquid Chromatography) | ≦ 1.2% |

### Example 3

### The Manufacture of C₁₂-C₁₈ Alkyltriethoxy-Dimethyl Amine Oxide Polycarboxylates.

To di-sodium maleate (216g, 1.35mol) dissolved in water (150g), C₁₂-C₁₈ alkyltriethoxy dimethyl amine oxide (180.5g, 0.5 mol) was added and nitrogen passed through the resulting mixture for 1 hour at 50°C, pH being 8.5
The deoxygenated mixture was heated to reflux and the initiator, tertiary butyl perbenzoate (126.7g, 0.65mol) was added dropwise in 3 aliquots over 12.5 hours.

The mixture was refluxed for a further 2.0 hours giving a reddish brown viscous product ¹H NMR showed 2.3 w/w unreacted maleate.

Reaction mixture was then treated with (38.3g) of 30 vol hydrogen peroxide to produce a light yellow clear mobile liquid of pH 7.0.

### Analysis

Dionex showed 1.02% w/w unreacted maleate.

The product was believed on the basis of NMR to comprise :-

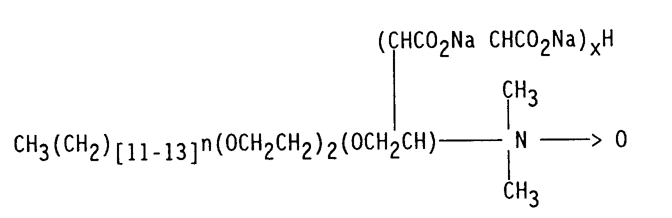

### Example 4

### The manufacture of C₁₂-C₁₄ Alkyl Ether Polycarboxylates using 3 mole Ether Carboxylate.

To di-sodium maleate (216g, 1.35 mol) dissolved in water (200g), C₁₂-C₁₄ sodium alkyl ether carboxylate (3EO) prepared from the mono carboxylic acid (192.7g 0.47 mol) was added, and N₂ passed through the resulting mixture for 1 hour at 50°C, pH being 7.5.

The deoxygenated mixture was heated to reflux and the initiator, tertiary butyl perbenzoate (120.3g, 0.62 mol) was added dropwise in 2 aliquots over 18.5 hours.

The mixture was then further refluxed for 8.5hrs giving a reddish brown liquid pH 7.0. Reaction mixture was then treated with 42.5g 30vol of hydrogen peroxide to produce to produce a light yellow liquid.

Dionex showed < 0.5% w/w unreacted maleate.

The product was believed on the basis of NMR to comprise :-

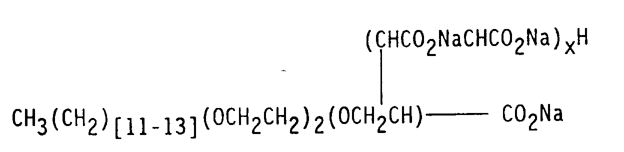

### Example 5

### The manufacture of C₁₂-C₁₄ Alkyl Ether Polycarboxylates using 3 mole Ether Carboxylic Acid.

To di-sodium maleate (162g, 1.01 mol) dissolved in water (289g), C₁₂-C₁₄ alkyl triethoxymono carboxylic acid (3EO) (146.4g, 0.375 mol) was added and nitrogen passed through the resulting mixture for 1 hour at 50°C.

The deoxygenated mixture was a colourless cloudy gel which was heated to reflux and the initiator, tertiary butyl perbenzoate (85.69g 0.44 mol) was added dropwise in 2 aliquots over 10.5 hours.

The mixture was then refluxed for a further 9 hours giving a dark brown viscose solution proton NMR showed <0.5% unreacted maleate.

Reaction mixture was then treated with 75g of 30vol. hydrogen peroxide, producing a light brown mobile liquid of pH 7.0

### Analysis

Free Maleate by dionex - <0.5% w/w

### Example 6

### The manufacture of C₁₂-C₁₄ Alkyl Ether Polycarboxylates Sulphate

To di-sodium maleate (194.4 1.22 mol) dissolved in water (358.1g), C₁₂-C₁₄ sodium alkyl ether sulphate (193.1g, 0.45 mol) was added, and nitrogen passed through the resulting mixture for 1 hour at 50°C.

The deoxygenated mixture was heated to reflux the initiator, tertiary butyl perbenzoate (98.82g 0.52 mol) was added dropwise in 4 aliquots over 12 hours.

The mixture was then refluxed for a further 19 hours giving a dark orange colour
Reaction mixture was then analysed to see if the sulphate group remained intact, by lead nitrate titration for free sodium sulphate using C₁₂-C₁₄ sodium alkyl ether sulphate as blank. The sodium sulphate group was found not to be liable under the conditions of the reaction.

Bleaching of the solution was then carried out. To 489.6g of product was added (24.48g) of 30 vol hydrogen peroxide at 85°C for 5 hours to give a light yellow liquid.

### Analysis

Proton NMR (after bleaching) <0.5%w/w free maleate
The product was believed to comprise :- 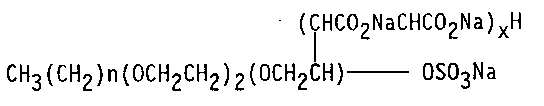
In the foregoing formulae it is assumed on the basis of the NMR that the maleate adds predominantly as dimaleate or polymaleate chains, on average one per polyether chain.

The available evidence does not exclude the possibility that some maleate adds as single molecules at several points in each polyether chain.

## Claims

1. A method for the manufacture of polycarboxylates which comprises reacting (i) a compound having a polyalkylenoxy chain and (ii) a water soluble salt of an unsaturated carboxylic acid, in aqueous solution in the presence of a free radical initiator.

2. A method according to claim 1 wherein said compound (i) is an alkyl or alkenyl polyalkoxylate.

3. A method according to claim 2 wherein said compound (i) is a C₁₀₋₂₀ alkyl or alkenyl polyethoxylate.

4. A method according to any foregoing claims wherein said salt (ii) is a salt of an unsaturated carboxylic acid having from 3 to 6 carbon atoms.

5. A method of preparing polycarboxylated polyalkyloxylates of general formula (I): 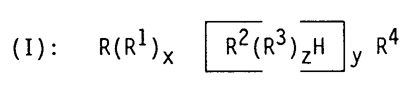 in which R is a straight or branched chain alkyl, alkaryl or alkenyl group or straight or branched chain alkyl or alkenyl carboxy group, having in each case up to 27 carbon atoms, or hydrogen, each R¹ is an OCH₂CH₂ or an OCH(CH₃)CH₂ group, each R² is an OC₂H₃ or OC₃H₅ group, each R³ is a C(R⁵)₂C(R⁵)₂ group, wherein from 1 to 4 R⁵ groups per R³ group are CO₂A groups, each other R⁵ group being a C₁-C₂ alkyl, hydroxyalkyl or carboxyalkyl group or H, R⁴ is OH, SO₄A, SO₃A, OR, sulphosuccinyl, OCH₂CO₂A or R⁶₂NR⁷, R⁶ is a C₁-C₄ alkyl or hydroxyalkyl group, R⁷ is a C₁-C₂₀ alkyl group, a benzyl group, a CH₂CO₂A, or -> 0 group or PO₄A₂, A is a cation capable of forming water soluble salts of said carboxylic acid, each z is from 1 to 5, y is at least 1 and (x+y) has an average value of from 1 to 50, wherein the R¹ and R² groups may be arranged randomly or in any order along the polyalkoxylate chain; which method comprises, reacting (i) a compound of the formula R(R¹)_{(x+y)}R⁴ wherein R, R¹ and R⁴ are as hereinbefore defined with (ii) a water soluble salt of an unsaturated carboxylic or polycarboxylic acid C(R⁵)₂ C(R⁵)₂, where each R⁵ is as defined above, in aqueous solution in the presence of a free radical initiator, at a temperature sufficient to produce said polycarboxylate.

6. A method according to claim 5 wherein said compound (i) comprises a poly R¹ group, (R¹)_{(x+y)} wherein said R¹ groups are ethyleneoxy groups with from 0 to 10% molar of the R¹ groups being optionally propylenoxy groups.

7. A method according to either of claims 5 and 6 wherein said R groups represents a mixture of predominantly C-10 to 20 alkyl groups.

8. A method according to claim 7 wherein said R group has an average of more than 11 carbon atoms.

9. A method for the production of polycarboxylated ether sulphates which comprises reacting a water soluble alkyl or alkenyl polyoxyalkylene sulphate with a water soluble salt of an unsaturated carboxylic acid in aqueous solution in the presence of a free radical initiator.

10. A method for the production of alkyl ether AEP which comprises reacting a water soluble alkyl or alkenyl polyoxyalkylene monocarboxylate with a water soluble salt of an unsaturated carboxylic acid in aqueous solution in the presence of a free radical initiator.

11. A method for the production of an polycarboxylated ether betaine which comprises reacting an alkyl or alkenyl polyoxyalkylene betaine with a water soluble salt of an unsaturated carboxylic acid in the presence of a free radical initiator.

12. A method according to any of claims 4 to 9 wherein said salt (ii) is a salt of an unsaturated carboxylic acid having from 3 to 6 carbon atoms.

13. A method according to claim 12 wherein said salt (ii) is a maleate, fumarate, acrylate, methacrylate, itaconate, crotonate, isocrotonate, angelate tiglate or mesaconate.

14. A method according to any foregoing claim wherein said salt (ii) is present in a proportion of from 0.2:1 to 5:1 molar, based on the average number of alkoxylate units per molecule in the compound (i).

15. A method according to any foregoing claim wherein the free radical initiator is tertiary butyl perbenzoate.

16. A method according to any foregoing claim wherein the water constitutes from 20 to 80% by weight of the total weight of the reaction mixture.

17. A method according to any foregoing claim wherein the pH of the reaction medium is from 5 to 10.

18. A method according to claim 17 wherein said pH is from 6 to 9.5.

19. An improved AEP which is substantially free from esters and which is soluble to give clear stable homogenous solutions at all concentrations up to 50% at a pH between 6.5 and 8, said AEPs having an average alkyl chain length of from 10 to 18 carbon atoms, and a polyether chain consisting of from 2 to 10 ethyleneoxy groups.

20. A novel surfactant having the formula (I) above wherein R is a C₆ to ₂₀ alkyl or alkenyl group or C₁₀ to ₂₀ alkyl or alkenyl carboxy group and R⁴ is a CH₂CO₂A or R⁶₂ NR⁷ group where R⁶, R⁷ and A have the same significance as in claim 2.

21. An N-C₆₋₂₀ alkyl or alkenyl 2 to 20 mole polyoxyalkylene-N,N-di C₁₋₄ alkylamine-N-oxide polycaroboxylate.

22. An N-C₆₋₂₀ alkyl or alkenyl 2 to 20 mole polyoxyalkylene-N-carboxymethyl amine polycarboxylate.

23. A polycarboxylated C₆₋₂₀ alkyl or alkenyl 2 to 20 mole polyoxyalkylene monocarboxylate.

24. A polycarboxylated C₆₋₂₀ alkyl or alkenyl 2 to 20 mole polyoxyalkylene sulphate.

25. A laundry detergent composition containing a surfactant according to any of claims 19 to 24.

26. A toiletry or cosmetic preparation containing a surfactant according to any of claims 19 to 24.
